# EUROPEAN PATENT APPLICATION

(11) **EP 1 878 466 A1**
(43) Date of publication of application: **16.01.2008**
(21) Application number: 06014266.8
(22) Date of filing: 10.07.2006
(51) Int. Cl.: A61N 5/06, A61H 23/02

(54) **Beauty treatment machine providing infrared light and vibration**

(71) Applicant: WinStone Technology Corp., Hsinchu City 300 (TW)
(72) Inventor: Zhan, Su-Yue, Hsinchu City 300 (TW)
(74) Representative: Schoppe, Fritz

(57) **Abstract**

A beauty machine is provided. The beauty machine includes a first case (4); a second case (7) assembled with the first case (4) and forming a containing space therebetween; a printed circuit board (6) disposed in the containing space; a vibrator (63) disposed on the printed circuit board (6) and providing a vibration; an emitter (64) electrically connected with the printed circuit board (6) and emitting a red light and a near infrared; a control circuit (65) disposed on the printed circuit board (6) and driving the vibrator (63) and the emitter (64); a fore case (1) retaining the first case (4) and the second case (7); and a far infrared device (8) disposed at a front part of the fore case (1).

## Description

The present invention relates to a beauty machine, in particular, to a beauty machine for simultaneously providing red light, near infrared ray and far infrared ray.

The love of beauty is a human nature. Therefore, the cosmetic market is gradually growing for years. At present, in the aspect of cosmetic devices, the most popular ones thereamong are the ultrasound importer, intense pulsed light (IPL) beauty device and anion importer, etc.

Currently, the physical vibrating principle is adopted by the present ultrasound importer in order to increase the size of gaps among cells, so as to facilitate the absorption of cosmetics by the human skin. However, it is also notable that not all kinds of cosmetics are importable into the skin through the physical vibration. However, even though the IPL beauty device is equipped with the function as to switch red light/blue light/infrared ray in sequence, it still lacks the functionality as to import anion and physical vibrations therein. Furthermore, though the anion importer is able to be used to import the cosmetics via the carrying of anions, it lacks the functionality as to diminish the molecules group by the photodynamic therapy, the vibration import and the far infrared ray. Hence, all of the aforementioned beauty devices possess only one single functionality. For the consumers, if one would like to simultaneously conduct the cosmetic by combining variety of the means of vibration import, the photodynamic therapy and the anion import, they must purchase the ultrasound importer, IPL beauty device and anion importer at the same time. This will be a finical burden for the consumers.

A phototherapy beauty device is disclosed in the Taiwanese Utility Model Patent No. 93206717, where a plurality of circularly arranged light-emitting diodes are simultaneously controlled in order to provide the therapy effect of omni-directional phototherapy. Furthermore, an ultrasound beauty device is disclosed in the Taiwanese Utility Model Patent No. 87108796 bearing the technical feature with respect to prevent the skin burning due to the over-heating metal detector. However, both of the aforementioned Patent Applications provide only one single function and also fail to provide an all-inclusive function integrating the corresponding prominent technical features possessed by the ultrasound importer, IPL beauty device and the anion importer simultaneously.

To overcome the mentioned drawbacks of the prior art, a novel beauty machinen is provided.

According to the first aspect of the present invention, a beauty machine is provided. The beauty machine includes a first case; a second case assembled with the first case and forming a containing space therebetween; a printed circuit board disposed in the containing space; a vibrator disposed on the printed circuit board and providing a vibration; an emitter electrically connected with the printed circuit board and emitting a red light and a near infrared; a control circuit disposed on the printed circuit board and driving the vibrator and the emitter; a fore case retaining the first case and the second case; and a far infrared device disposed at a front part of the fore case.

Preferably, the beauty machine further includes a power socket disposed on the printed circuit board for receiving a direct current from one of a transformer and a USB (Universal Serial Bus).

Preferably, the beauty machine further includes a waterproof seal disposed around the power socket for resisting liquid.

Preferably, the beauty machine further includes a power switch disposed on the printed circuit board and controlling the direct current.

Preferably, the beauty machine further includes a rubber cover covering on the power switch, protruding from the first case, so that the power switch is controlled by pushing the rubber cover.

Preferably, the first case further includes an opening for protruding the rubber cover therefrom.

Preferably, the first case has a concave thereon for placing a recognizer and a shell therein.

Preferably, the recognizer is a sticker.

Preferably, one of a logotype and a pattern is printed on the recognizer.

Preferably, the shell is disposed on the recognizer for protecting the recognizer.

Preferably, the shell is transparent.

Preferably, the vibrator is used to provide a massage effect via a physical vibration.

Preferably, the emitter comprises a red light LED (Light-Emitting Diode) and a near infrared LED.

Preferably, the red light LED emits the red light and the near infrared LED emits the near infrared.

Preferably, the red light and the near infrared are alternately emitting.

Preferably, the fore case and the far infrared device are formed integratedly via an injection-molding.

Preferably, the fore case is plastic.

Preferably, the far infrared device is a far infrared gem.

Preferably, the far infrared gem is one selected from a group consisting of an aluminum oxide, a zirconium oxide, a titania, a yttrium trioxide and a rare thorium mineral.

Preferably, the beauty machine further includes a heating device disposed on the far infrared gem and connected with the control circuit.

Preferably, the beauty machine further includes a Li battery for providing electric power to the beauty machine and connected with a contactless battery charger.

Preferably, the beauty machine further includes a display being an OLED (organic light emitting diode) in order to display the temperature, the remaining battery power, the remaining treatment and the warning.

According to the second aspect of the present invention, a beauty machine is provided. The beauty machine includes a first case; a second case assembled with the first case and forming a containing space therebetween; a printed circuit board disposed in the containing space; a vibrator disposed on the printed circuit board and providing a vibration; an emitter electrically connected with the printed circuit board and emitting a red light and a near infrared; a control circuit disposed on the printed circuit board and driving the vibrator and the emitter; a fore case retaining the first case and the second case; and a far infrared gem disposed at a front part of the fore case, wherein the far infrared gem is one selected from a group consisting of an aluminum oxide, a zirconium oxide, a titania, a yttrium trioxide and a rare thorium mineral.

According to the third aspect of the present invention, a beauty machine is provided. The beauty machine includes a case having a first end; a circuit mounted in the case; an emitter electrically connected with the circuit and emitting a red light and a near infrared; and a far infrared device mounted at the first end.

According to the fourth aspect of the present invention, a beauty machine is provided. The beauty machine includes a case having a first end; a circuit mounted in the case; a vibrator electrically connected with the circuit and providing a vibration; an emitter electrically connected with the circuit and emitting a red light and a near infrared; and a far infrared device mounted at the first end.

The foregoing and other features and advantages of the present invention will be more clearly understood through the following descriptions with reference to the drawings, wherein:

Fig. 1 is an exploded view of the beauty machine according to a preferred embodiment of the present invention;

Figs. 2(a)~2(d) are schematic diagrams showing the appearance of the beauty machine according to a preferred embodiment of the present invention; and

Fig. 3 is a schematic diagram showing the beauty machine in use according to a preferred embodiment of the present invention.

The present invention will now be described more specifically with reference to the following embodiments. It is to be noted that the following descriptions of preferred embodiments of this invention are presented herein for the aspects of illustration and description only; it is not intended to be exhaustive or to be limited to the precise form disclosed.

There are visible light, and invisible infrared ray and ultraviolet ray in the sunlight. The electromagnetic wave produced by the infrared ray is identical with that produced by the visible light, but the wavelength thereof exceeds the ordinary visible red light. About 59% of the sunlight is constituted by the infrared ray that is technically categorized into four types: the near infrared ray, the intermediate infrared, the far infrared ray and the extra far infrared ray. When the near infrared ray is projected onto a specific spot of the human body, the projected spot will sense scorched. On the contrary, the far infrared ray doesn't stir the human body, but enhances the body energy, thereby warming up the human body. Besides, the organs of the human body are resonated with the far infrared ray, which amounts to the therapy effect.

The infrared ray is essentially one kind of electromagnetic wave. It is capable for any object to release the infrared ray while the temperature thereof is higher than 0°K (absolute temperature). The infrared ray existing in the range of 0.75~1000 µm is further categorized into three types according to the energy contained therein: the near infrared ray (the wavelength thereof is 0.75 - 1.5 µm), the intermediate infrared ray (the wavelength thereof is 1.5 ~ 5.6 µm) and the far infrared ray (the wavelength thereof is 5.6 ~ 1000 µm).

There are three technical features for the infrared ray:

The first technical feature is the radiation nature. The propagation of infrared ray does not need the assistance of medium, e.g. the atmosphere. The infrared ray is directly conducted to the object, which is radioactive as the sunlight.

The second technical feature is the infiltration characteristic. It is feasible for the infrared ray to penetrate into the subcutaneous issue to warm up the human body and activate human cells.

The third technical feature is the resonance and absorption characteristic. The infrared ray is intrinsically the light and electromagnetic wave. Since the wavelength of infrared ray is approximate to that emitted from the human body (about 4-50 µm), the molecules existing inside the human body will be resonated once accepting the infrared ray. Therefore, the human body will absorb the energy containing in the infrared ray and collaterally the entire physiological faculty of human body is activated. Moreover, there are varieties of organic chemical compounds existing in the human body, and the organism thereof is unable to be normally performed without the infrared ray. On the other hand, if each portion of the human body sufficiently absorbs the infrared ray, the respective molecules thereof will be resonated with the infrared ray, through which the water molecule is activated.

When the frequency of the physical vibration of organic molecules harmonizes with that of the infrared ray, the resonance is happened therebetween, and the vibrating amplitude of molecules will be increased. The aforementioned is the essential theorem with respect to the resonance absorption effect and heat-generating phenomenon for the infrared ray. The resonance absorption effect is enhanced by the coherent vibrating among atoms and molecules so that consequently the temperature under human skin is raised, the capillaries are simultaneously expanded, the blood is accelerated and the blood stasis is obviated thererfrom after the resonated physical vibration, which amounts to further facilitate the blood circulation and the metabolism of cells.

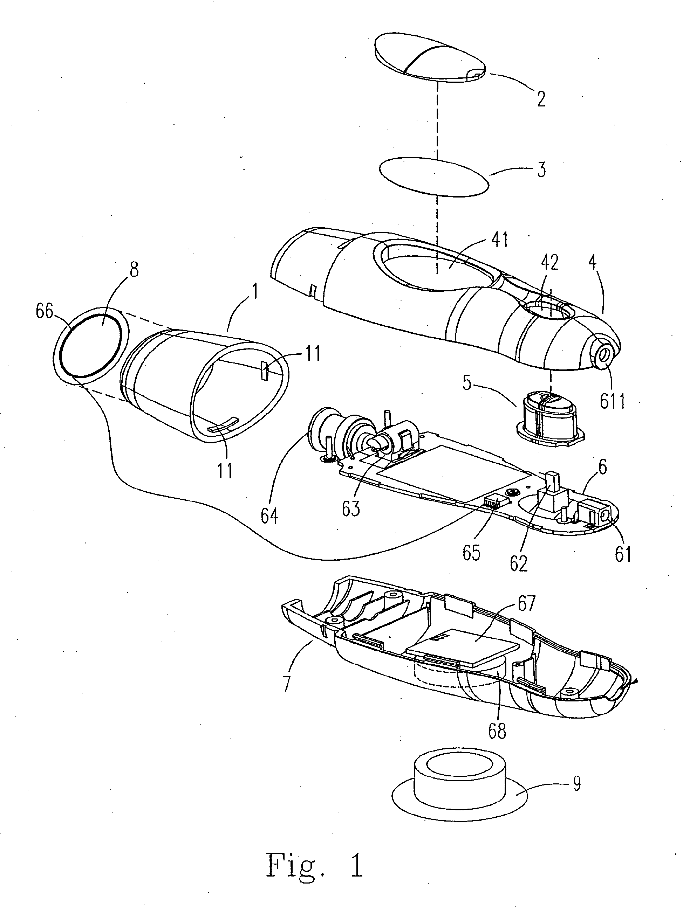

Please refer to Fig. 1, which is a exploded view of the beauty machine according to a preferred embodiment of the present invention. The beauty machine includes an upper case 4, a lower case 7, a printed circuit board 6, a fore case 1 and a far infrared gem 8, wherein a containing space is formed between the lower case 7 and the upper case 4 for disposing the printed circuit board 6. The fore case 1 and the far infrared gem 8 are formed integratedly via an injection-molding. Further, the fore case 1 has four wedges 11 for retaining the upper case 4 and the lower case 7 when the upper case 4 and the lower case 7 are assembled. Preferably, the fore case 1 is plastic.

The upper case 4 has a concave 41 for placing a sticker 3 and a shell 2 therein, wherein one of a logotype and a pattern is printed on the sticker 3. Each of the front and back sides of the sticker 3 has the glue thereon, and the shell 2 is adhered to the sticker 3 with the glue at the front side while the upper case 4 is adhered to the sticker 3 with the glue at the back side. The shell 2 is disposed on the sticker 3 for protecting the sticker 3. Preferably, the material of the shell 2 is a transparent acrylic.

There are a power socket 61, a waterproof seal 611, a power switch 62, a vibrator 63, an emitter 64, a control circuit 65, a heating device 66, a Li battery 67 and a contactless battery charger 68 disposed on the printed circuit board 6. Additionally, a power supplier 9 of the contactless battery charger 68 is placed at an adequate position so as to provide an electromagnetic exchange field to the contactless battery charger 68. The power socket 61 is used to connect with one of a transformer (not shown) and a USB (Universal Serial Bus) for providing a direct current to the beauty machine, and a waterproof seal 611 is disposed around the power socket for resisting liquid. It is noted that the standard of the power socket implemented on the power supplier 9 of the contactless battery charger 68 is identical with that of the power socket 61. The power switch 62 is used to control the direct current. A pair of electrically conductive pieces under the vibrator 63 is connected with the conducting wires of the printed circuit board 6 for providing a high frequency vibration so as to achieve a massage effect via a physical vibration. The vibrator 63 is driven by the control circuit 65. The vibrator is vibrated in a simulate pulse way so as to substitute the function of the ultrasound importer.

The emitter 64 has a red light LED (Light-Emitting Diode) and a near infrared LED. When the emitter 64 turns red, the emitter 64 is emitting the red light, whereas when the emitter 64 turns off, the emitter 64 is emitting the invisible near infrared ray. Hence, the red light and the near infrared ray are alternately emitting and propagating to the user's skin via the semi-transparent far infrared gem 8. The infrared ray possesses the effect of anti-inflammation and can cure slight acne. The near infrared ray can facilitate the recovery of wounds and the repair of cells. The control circuit 65 is used to produce a pulse oscillation wave to drive the vibrator 63 and the emitter 64.

Furthermore, a heating device 66 is disposed on the far infrared gem 8 and is electrically connected with the control circuit 65. The Li battery 67 is electrically connected with the contactless battery charger 68 for providing electric power to the beauty machine. Besides, a display such as an OLED (organic light emitting diode) is disposed on the beauty machine in order to display the temperature, the remaining battery power, the remaining treatment and the warning.

Besides, the beauty machine of the present invention further includes a rubber cover 5, which is hollow and soft, covers the power switch 62 and protrudes from an opening 42 of the first case 4, so that the power switch 62 is controlled by pushing the rubber cover 5.

Furthermore, the material of the far infrared gem 8 is one selected from a group consisting of an aluminum oxide, a zirconium oxide, a titania, a yttrium trioxide and a rare thorium mineral. The far infrared gem 8 is able to produce the far infrared ray with high-level energy (radiation rate: 0.936) when the surrounding temperature is around 36°C. On one hand, the far infrared ray resonates with the molecules of the cosmetic, which renders the volume of the molecule group of the cosmetic compacted so that the compacted cosmetic is much easier to be infiltrated into and absorbed by the human skin. On the other hand, the quasi acupuncture effect of the infrared ray will expedite the expansion of the capillaries so that the infiltrated cosmetic is able to be quickly absorbed. The absorption rate of the coating type cosmetic is ordinarily lower than 5%, but it is promoted through the beauty device of the present invention. Moreover, the far infrared ray further owns the following effects with respect to beauty treatment:

(1) Facilitating sweat and to eliminate the subcutaneous fat: purge the sweat gland, sebum and subcutaneous fat, and eliminate the filth.

(2) Promoting the physiological oxidation-reduction reaction: reduce the accumulation of extra fat.

(3) Activate the physiological mechanism and function.

(4) Agitating the hormone and enzyme: eliminate the heavy metal and the deposition of the poisonous substance so that the nutrition can be efficiently delivered to the skin cells.

(5) Recovering: eliminate the blood stasis and scars, and recover the muscle mechanism and consciousness.

(6) Treating the sundry beauty issues: open the pore to provide the fresh oxygen, assuage the breathing, and make the skin smooth and tender.

Besides, the far infrared gem 8 is able to be easily replaced. The user can replace the far infrared gem 8 with a new one having a different size according to actual needs. The replacement is achievable in a very short time and quite convenient. The most important is that the user doesn't need to replace the main body of the beauty machine.

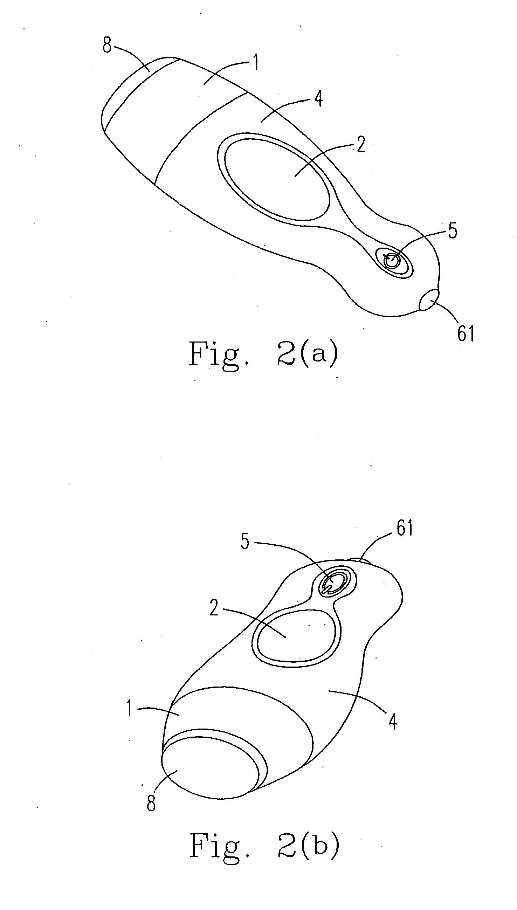

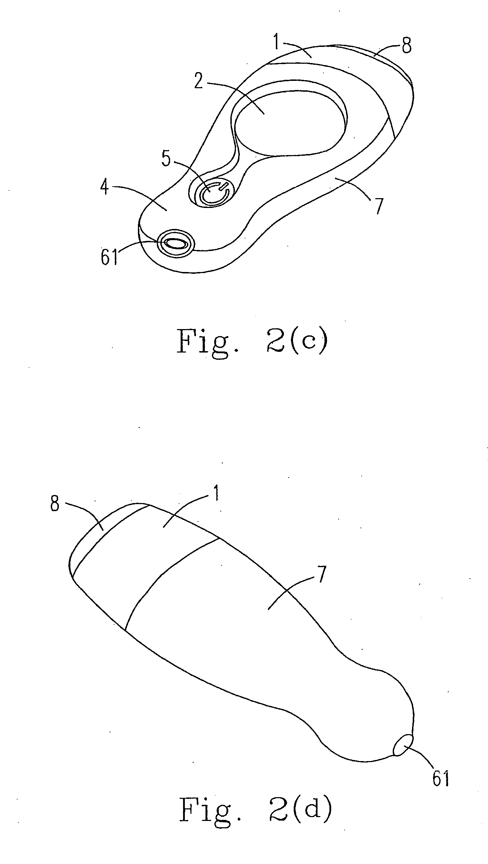

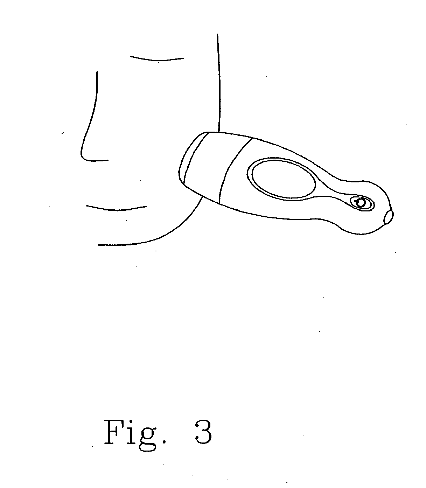

Please refer to Figs. 2(a)~2(d), which are schematic diagrams showing the appearance of the beauty machine according to a preferred embodiment of the present invention. Please refer to Fig. 3, which is a schematic diagram showing the beauty machine in use according to a preferred embodiment of the present invention.

To sum up the aforementioned, the present invention not only provides the physical vibration to import the cosmetic into the human skin, but also provides anions while the far infrared gem 8 is vibrating. Since the anions are incompatible with the cosmetic carrying the negative electrons, the anions are imported into the human skin thereby. Furthermore, the red light in the present invention is able to promote the synthesis of the collagen, facilitate the release of the ATP energy storing in cells, promote the synthesis of DNA and RNA, improve the effect of the processing mechanism for phagocyte and the effect of anti-inflammation, which results in the fine and prominent beauty effect on the human skin. Moreover, the near infrared ray is able to diminish the size of sebaceous gland and therefore possesses the dependent effect on preventing the acne. Hence, the present invention provides a beauty machine which simultaneously provides the red light, near infrared ray and far infrared ray and further includes the ultrasound import function. The beauty machine integrates diverse functionalities separately existing in the present beauty machines and efficiently improves the defects existing in the prior arts, which results in the industrial merits. In this manner, the objective of the present invention is achieved and the skilled person is able to implement the beauty machine on the basis of the aforementioned disclosures. Therefore, the present invention not only has novelty and progressiveness, but also has utility.

## Claims

1. A beauty machine, **characterized in that** it comprises:
a first case (4);
a second case (7) assembled with the first case (4) and forming a containing space therebetween;
a printed circuit board (6) disposed in the containing space;
a vibrator (63) disposed on the printed circuit board (6) and providing a vibration;
an emitter (64) electrically connected with the printed circuit board (6) and emitting a red light and a near infrared;
a control circuit (65) disposed on the printed circuit board (6) and driving the vibrator (63) and the emitter (64);
a fore case (1) retaining the first case (4) and the second case (7); and
a far infrared device (8) disposed at a front part of the fore case(1).

2. The beauty machine according to claim 1, **characterized in that** it further comprises a power socket disposed on the printed circuit board for receiving a direct current from one of a transformer and a USB (Universal Serial Bus).

3. The beauty machine according to claim 2, **characterized in that** it further comprises a waterproof seal disposed around the power socket for resisting liquid.

4. The beauty machine according to claim 1, **characterized in that** it further comprises a power switch disposed on the printed circuit board and controlling the direct current.

5. The beauty machine according to claim 4, **characterized in that** it further comprises a rubber cover covering on the power switch, protruding from the first case, so that the power switch is controlled by pushing the rubber cover.

6. The beauty machine according to claim 5, **characterized in that** the first case further comprises an opening for protruding the rubber cover therefrom.

7. The beauty machine according to claim 1, **characterized in that** it comprises the first case having a concave thereon for placing a recognizer and a shell therein.

8. The beauty machine according to claim 7, **characterized in that** the recognizer is a sticker.

9. The beauty machine according to claim 7, **characterized in that** one of a logotype and a pattern is printed on the recognizer.

10. The beauty machine according to claim 7, **characterized in that** the shell is disposed on the recognizer for protecting the recognizer.

11. The beauty machine according to claim 10, **characterized in that** the shell is transparent.

12. The beauty machine according to claim 1, **characterized in that** the vibrator is used to provide a massage effect via a physical vibration.

13. The beauty machine according to claim 1, **characterized in that** the emitter comprises a red light LED (Light-Emitting Diode) and a near infrared LED.

14. The beauty machine according to claim 13, **characterized in that** the red light LED emits the red light and the near infrared LED emits the near infrared.

15. The beauty machine according to claim 1, **characterized in that** the red light and the near infrared are alternately emitting.

16. The beauty machine according to claim 1, **characterized in that** the fore case and the far infrared device are formed integratedly via an injection-molding.

17. The beauty machine according to claim 1, **characterized in that** the fore case is plastic.

18. The beauty machine according to claim 1, **characterized in that** the far infrared device is a far infrared gem.

19. The beauty machine according to claim 18, **characterized in that** the far infrared gem is one selected from a group consisting of an aluminum oxide, a zirconium oxide, a titania, a yttrium trioxide and a rare thorium mineral.

20. The beauty machine according to claim 19, **characterized in that** it further comprises a heating device disposed on the far infrared gem and electrically connected with the control circuit.

21. The beauty machine according to claim 1, **characterized in that** it further comprises a Li battery connected with a contactless battery charger for providing electric power to the beauty machine.

22. The beauty machine according to claim 1, **characterized in that** it further comprises a display being an OLED (organic light emitting diode) in order to display the temperature, the remaining battery power, the remaining treatment and the warning.

23. A beauty machine, **characterized in that** it comprises:
a first case (4);
a second case (7) assembled with the first case (4) and forming a containing space therebetween;
a printed circuit board (6) disposed in the containing space;
a vibrator (63) disposed on the printed circuit board (6) and providing a vibration;
an emitter (64) electrically connected with the printed circuit board (6) and emitting a red light and a near infrared;
a control circuit (65) disposed on the printed circuit board (6) and driving the vibrator (63) and the emitter (64);
a fore case (1) retaining the first case (4) and the second case (7); and
a far infrared gem (8) disposed at a front part of the fore case (1), wherein the far infrared gem (8) is one selected from a group consisting of an aluminum oxide, a zirconium oxide, a titania, a yttrium trioxide and a rare thorium mineral.

24. A beauty machine, **characterized in that** it comprises:
a case (1, 2, 4, 7) having a first end;
a circuit (6, 65) mounted in the case (1, 2, 4, 7);
an emitter (64) electrically connected with the circuit (6, 65) and emitting a red light and a near infrared; and
a far infrared device (8) mounted at the first end.

25. A beauty machine, **characterized in that** it comprises:
a case (1, 2, 4, 7) having a first end;
a circuit (6, 65) mounted in the case (1, 2, 4, 7);
a vibrator (63) electrically connected with the circuit (6, 65) and providing a vibration;
an emitter (64) electrically connected with the circuit (6, 65) and emitting a red light and a near infrared; and
a far infrared device (8) mounted at the first end.
